Europäisches Patentamt

European Patent Office

Office européen des brevets

(19)

(11) **EP 0 586 740 B1**

(12) **EUROPÄISCHE PATENTSCHRIFT**

(45) Veröffentlichungstag und Bekanntmachung des Hinweises auf die Patenterteilung:
**18.12.1996 Patentblatt 1996/51**

(51) Int. Cl.$^6$: **A61M 5/142**, A61M 5/36

(21) Anmeldenummer: **92115568.5**

(22) Anmeldetag: **11.09.1992**

(54) **Vorrichtung zum Zurückhalten von Luftblasen**

Device for preventing passage of air bubbles

Dispositif pour éviter le passage des bulles d'air

(84) Benannte Vertragsstaaten:
**DE FR GB IT NL SE**

(43) Veröffentlichungstag der Anmeldung:
**16.03.1994 Patentblatt 1994/11**

(73) Patentinhaber:
• **Siemens-Elema AB**
**171 95 Solna 1 (SE)**
Benannte Vertragsstaaten:
**SE**

• **SIEMENS AKTIENGESELLSCHAFT**
**80333 München (DE)**
Benannte Vertragsstaaten:
**DE FR GB IT NL**

(72) Erfinder: **Slettenmark, Bruno**
**S-175 60 Järfälla (SE)**

(56) Entgegenhaltungen:
**EP-A- 0 142 866**     **US-A- 4 360 019**
**US-A- 4 900 312**

Anmerkung:   Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft eine Vorrichtung zum Flüssigkeitstransport, inbesondere ein Medikamentendosiergerät, mit einer intermittierend arbeitenden Pumpe und einem dieser eingangsseitig vorgeordneten Filter zum Zurückhalten von Luftblasen, das derart dimensioniert ist, daß Luftblasen mit einem größeren Durchmesser als ein vorgegebener Mindestdurchmesser an einem freien Passieren des Filters gehindert werden und daß der bei einer Pumpaktion von der Flüssigkeitsströmung durch das Filter erzeugte maximale Druckabfall geringer ist, als der zum Fördern einer Luftblase mit einem größeren Durchmesser als dem vorgegebenen Mindestdurchmesser durch das Filter erforderliche Druck.

Bei einer derartigen aus der US-A-4 604 090 (Patentfamilienmitglied der EP-A-0 142 866) bekannten Vorrichtung handelt es sich um ein in einem lebenden Organismus implantierbares Medikamentendosiergerät, in dem eine durch elektrische Impulse angesteuerte elektromagnetisch betätigbare Balgenpumpe bei jedem Impuls eine dem Hubvolumen der Pumpe entsprechende Menge eines flüssigen Medikaments aus einem Medikamentenspeicher heraussaugt und einem Katheter zuführt, der die Medikamentenportionen an eine geeignete Stelle im Organismus transportiert und dort injiziert. Die bei der bekannten Vorrichtung vorgesehene Balgenpumpe weist ein relativ großes Totvolumen auf, so daß für den Fall, daß Luft in die Pumpe gelangt, die Medikamentenförderleistung der Pumpe mit zunehmender Luftansammlung abnimmt und schließlich die Flüssigkeitsförderung völlig zum Erliegen kommt, weil bei jeder Pumpaktion nur noch die in der Pumpe angesammelte Luftmenge komprimiert wird. Da in der zu pumpenden Flüssigkeit immer eine gewisse Gasmenge gelöst ist und beim Füllen des Medikamentenspeichers unter normalen Bedingungen Luft in den Medikamentenspeicher gelangen kann, wenn nicht aufwendige Gegenmaßnahmen wie Entgasung der Flüssigkeit oder Füllen des Medikamentenspeichers im Vakuum getroffen werden, ist bei der bekannten Vorrichtung der Pumpe ein Filter zum Zurückhalten von Luftblasen vorgeordnet. Das Filter besteht im wesentlichen aus einer flexiblen Filterscheibe in Form einer porösen Membran oder eines Maschengitters mit einer Porenweite von 0,22 bis 5 µm bzw. Maschenweite von z.B. 15 µm, so daß nur extrem kleine Luftblasen mit gleichem oder kleinerem Durchmesser das Filter ungehindert passieren können. Wegen der äußerst geringen Poren- bzw. Maschenweite des Filter kann jedoch bereits eine geringe Flüssigkeitsströmung durch das Filter zu einem derart großen Druckabfall über dem Filter führen, daß dadurch auch größere Luftblasen durch das Filter gedrückt werden. Aus diesem Grund ist bei der bekannten Vorrichtung die Filterscheibe so dünn und damit so flexibel ausgebildet, daß die Filterscheibe bei jeder Pumpaktion durch den von der Pumpe erzeugten Saugdruck ausgelenkt wird und danach langsam in ihre Ausgangsposition zurückkehrt, wodurch die Flüssigkeitsströmung und damit der Druckabfall durch das Filter begrenzt wird.

Wegen der äußerst geringen Poren- bzw. Maschenweite des Filters besteht bei der bekannten Vorrichtung die Gefahr, daß die Poren bzw. Maschen durch Fremdpartikel oder Ausfällungen des Medikaments (z.B. Insulin) sehr schnell verstopfen, wodurch je nach Flexibilität der Filterscheibe entweder der Druckabfall auf Grund der Flüssigkeitsströmung durch die noch nicht verstopften Poren oder Maschen so groß wird, daß Luftblasen durch das Filter hindurch gedrückt werden, oder das Filter nicht mehr in seine Ausgangslage zurückkehrt, wodurch die Förderleistung der Pumpe verringert wird. Auf der anderen Seite ist aber eine Vergrößerung der Poren- bzw. Maschenweite ausgeschlossen, weil dies zu einer erhöhten Luftdurchlässigkeit des Filters führen würde, was jedoch auf Grund des oben erwähnten relativ großen Totvolumens der bekannten Pumpe unbedingt zu vermeiden ist.

Wird das Filter bei der bekannten Vorrichtung auf seiner von der Pumpe abgewandten Seite teilweise durch die sich ansammelnde Luft abgedeckt, so führt dies zu denselben Auswirkungen, wie bei der oben beschriebenen Verstopfung der Filterporen bzw. -maschen. Hinzu kommt, daß die Poren bzw. Maschen auf Luftblasen wie Potentialmulden wirken, in denen sich die Luftblasen festsetzen und die betreffenden Poren oder Maschen verstopfen. Wenn das bekannte Filter schließlich völlig durch Luftblasen verstopft ist oder von einer großen Luftblase bedeckt ist, kommt die Flüssigkeitsförderung völlig zum Erliegen.

Der Erfindung liegt daher die Aufgabe zugrunde, bei einer Vorrichtung zum Flüssigkeitstransport mit einer intermittierend arbeitenden Pumpe auf effektive Weise zu verhindern, daß die Flüssigkeitsförderung durch die Pumpe auf Grund von Luftblasen in der zu fördernden Flüssigkeit zum Erliegen kommt.

Gemäß der Erfindung wird die Aufgabe dadurch gelöst, daß bei der Vorrichtung der eingangs angegebenen Art das Filter eine Durchlaßöffnung in Form eines langen Spalts enhält und daß das Volumen des Spalts größer als das Fördervolumen der Pumpe bei einer einzelnen Pumpaktion ist. Selbst wenn der Spalt nahezu völlig mit einer Luftblase bedeckt ist, kann diese den Spalt nicht passieren, solange nur der Spalt noch an irgend einer Stelle in Kontakt mit der Flüssigkeit steht. Bei einer Pumpaktion wird die Luftblase in den Spalt hineingesaugt, ohne diesen jedoch zu passieren, weil das Volumen des Spaltes größer ist, als das Fördervolumen der Pumpe bei der Pumpaktion. Unmittelbar nach der Pumpaktion geht die Grenzfläche zwischen der Luftblase und der Flüssigkeit auf Grund der Oberflächenspannung der Flüssigkeit wieder in ihre Ausgangslage vor der Pumpaktion zurück, wobei sich der Spalt wieder mit der Flüssigkeit füllt, welche von der von der Pumpe abgewandten Seite des Filters in den Spalt gelangt.

Bei einem herkömmlichen Filter mit Poren oder Maschen ist dies nicht möglich, weil die Poren bzw.

Maschen voneinander isoliert sind. Angenommen, daß z.B. 99 von 100 Poren mit Luftblasen bedeckt sind und daß die Luftblasen bei einer Pumpaktion alle gleichmäßig in die betreffenden Poren hineingesaugt werden, wobei dann das Gesamtvolumen der Luftfüllung der Poren etwa 99% des Pumpvolumens bei der Pumpaktion ausmacht, so müßte bei einer Wiederauffüllung der 99 Poren mit der Flüssigkeit die entsprechende Menge von 99% des Pumpvolumens durch die einzige freie Pore gefördert werden. Der dabei über diese einzige Pore entstehende Druckabfall der strömenden Flüssigkeit müßte durch die Oberflächenspannung der Luftblasen über den anderen 99 Poren aufgebracht werden, was nicht möglich ist. Davon abgesehen können Poren und Maschen nicht mit einer solchen Genauigkeit hergestellt werden, daß sie über das gesamte Filter jeweils gleich groß sind. Wird daher auf Grund einer teilweisen Luftbedeckung eines herkömmlichen Filters der Strömungsdruck durch die unbedeckten Poren so groß, daß er den Kapillardruck der Luftblase über der jeweils größten luftbedeckten Pore erreicht, so wird die Luftblase durch diese Pore hindurchgefördert, da das Volumen der Pore nur ein Bruchteil des Pumpvolumens bei einer Pumpaktion ist. Ein Porenvolumen einer einzelnen Pore so groß wie das Pumpvolumen ist jedoch nicht realisierbar, da entweder die Porenweite so groß gemacht werden müßte, daß auch große Luftblasen ungehindert passieren können, oder die Porentiefe müßte so groß werden, daß der Strömungswiderstand für die Flüssigkeit und damit der Druckabfall über den Filter extreme Werte erreichen würde.

Demgegenüber kann bei der erfindungsgemäßen Vorrichtung allein über die Spaltlänge das Spaltvolumen größer als das Pumpvolumen bei einer Pumpaktion dimensioniert werden, ohne daß ungünstige Kompromisse für die Bemessung der Spaltbreite im Hinblick auf den Durchmesser der zurückzuhaltenden Luftblasen und die Dimensionierung der Spalttiefe im Hinblick auf den von der Flüssigkeitsströmung erzeugten Druckabfall getroffen werden müssen. Da bei der erfindungsgemäßen Vorrichtung das im wesentlichen über die Spaltlänge einstellbare Spaltvolumen für das Zurückhalten von Luftblasen maßgeblich ist, während bei einem herkömmlichen Porenfilter wegen des äußerst geringen Porenvolumens der Porendurchmesser das Luftzurückhaltevermögen des Filters bestimmt, sind die Anforderungen an die absolute Genauigkeit bei der Herstellung des erfindungsgemäßen Filters weitaus geringer als bei herkömmlichen Poren- oder Maschenfiltern.

Schließlich ist die Verstopfungsgefahr durch Partikel oder Ausfällungen der Flüssigkeit bei einem Spalt geringer als bei einem Poren- oder Maschenfilter. Insbesondere Insulinlösungen und andere, Proteine enthaltene Flüssigkeiten neigen zur Adsorption und Bildung von Oberflächenbelägen. Da der von einer Flüssigkeitsströmung durch ein Filter erzeugte Druckabfall bei einem Porenfilter mit einem Faktor $s_p^{-4}$ vom Porendurchmesser $s_p$, dagegen bei einem Spalt nur mit einem Faktor $s_s^{-3}$ von der Spaltbreite $s_s$ abhängig ist, nimmt der Strömungswiderstand bei einer Verengung von Poren weitaus schneller zu als bei einer Verengung des Spalts.

Natürlich können bei der erfindungsgemäßen Vorrichtung erforderlichenfalls anstelle des einen Spaltes zwei oder mehrere Spalte mit gleicher Gesamtlänge vorgesehen werden, jedoch ist auf Grund der oben genannten Vorteile ein einziger Spalt vorzuziehen. Um zu erreichen, daß bei der erfindungsgemäßen Vorrichtung die Grenzfläche zwischen der Luftblase und der Flüssigkeit nach jeder Pumpaktion auf Grund der Oberflächenspannung der Flüssigkeit wieder in ihre Ausgangslage vor der Pumpaktion zurückkehrt und sich der Spalt wieder mit der Flüssigkeit füllt, ist vorzugsweise vorgesehen, daß das Filter zumindest in der Umgebung des Spaltes aus einem von der Flüssigkeit benetzbaren Material besteht. Ein Beispiel hierfür ist reines Titan, das von in Wasser gelöstem Insulin als zu fördernder Flüssigkeit benetzt wird und sich dieser gegenüber inherent verhält. Alternativ oder ergänzend kann der Flüssigkeit ein Benetzungsmittel zugesetzt sein.

Um so weit wie möglich zu verhindern, daß der Spalt völlig von einer Luftblase bedeckt wird, weist dieser eine möglichst weite Ausstreckung in eindimensionaler oder vorzugsweise zweidimensionaler Richtung auf. Dabei ist der Spalt, auch aus herstellungstechnischen Gründen, bevorzugt als Ringspalt ausgebildet.

Entsprechend einer vorteilhaften Weiterbildung der erfindungsgemäßen Vorrichtung ist vorgesehen, daß das Filter auf seiner von der Pumpe abgewandten Seite und/oder eine daran angrenzende Innenwandung eines die zu pumpende Flüssigkeit aufnehmenden Behälters eine Ausformung aufweisen, die verhindert, daß eine Luftblase im Bereich des Filters eine stabile Gleichgewichtslage einnimmt. Dazu kann das Filter auf seiner von der Pumpe abgewandten Seite und/oder die daran angrenzende Innenwand des Behälters im wesentlichen konvex ausgebildet sein. So ist insbesondere bei einem quer über eine konvexe Oberfläche verlaufenden langgestreckten Spalt die Gefahr einer völligen Luftbedeckung des Spaltes unabhängig von der zufälligen Lage der Oberfläche minimal. Dadurch kann sich in der zu fördernden Flüssigkeit Luft bis zu einer vorgegebenen Menge ansammeln, ohne daß das Filter von der Luftmenge völlig bedeckt werden kann. Handelt es sich bei der erfindungsgemäßen Vorrichtung um ein implantierbares Medikamentendosiergerät, so ist dieses derart ausgebildet, daß die vorgegebene Luftmenge um einen Sicherheitsbetrag größer ist, als diejenige Luftmenge, die bei einer normal verlaufenden Füllung des Medikamentenspeichers in diesen gelangen kann. Wenn vor jeder Wiederfüllung des Medikamentenspeichers dieser zunächst entleert wird und der Sicherheitsbetrag größer gewählt ist, als ein Restvolumen, unterhalb dessen eine weitere Entleerung des Medikamentenspeichers technisch nicht möglich ist, so ist im Normalfall ausgeschlossen, daß das Filter völlig von einer Luftblase bedeckt werden kann.

Sobald der Fall eintritt, daß der Spalt des erfindungsgemäßen Filters vollständig mit einer Luftblase bedeckt ist, wird keine Flüssigkeit mehr durch das Filter hindurchgepumpt. Je nach Dimensionierung des Filters und der Pumpe kann nun vorgesehen werden, daß entweder die Luftblase durch das Filter und die Pumpe hindurchgepumpt wird, um im Anschluß daran die Förderung der Flüssigkeit wieder aufzunehmen, oder daß das Filter die Luftblase gegen den Saugdruck der Pumpe zurückhält und damit die Flüssigkeitsförderung solange zum Erliegen kommt, bis der Spalt des Filters Kontakt mit der Flüssigkeit bekommt. Um wie z.B. bei der Infusion von Insulin zunächst die Luftblase und danach wieder das Insulin durch das Filter und die Pumpe hindurchpumpen zu können, kann bei der erfindungsgemäßen Vorrichtung die Pumpe eine Kolbenpumpe mit einem weitestgehend totraumfreien Hubraum sein. Solche Kolbenpumpen sind beispielsweise aus der EP-A-0 259 668 und der EP-A-0 287 920 bekannt und können wegen ihres vernachlässigbar geringen Totraumvolumens Gasblasen auch bei hoher Druckdifferenz zwischen der Eingangs- und Ausgangsseite der Pumpe pumpen.

Soll dagegen wie z.B. bei Infusionen in die Blutbahn jeglicher Transport von Luft ausgeschlossen werden, so genügt es im Prinzip, die Pumpe und den Spalt im Verhältnis zueinander so zu dimensionieren, daß der von der Pumpe erzeugte Saugdruck nicht ausreicht, die den Spalt des Filters bedeckende Luftblase durch den Spalt hindurch zu fördern. Da jedoch andererseits der Saugdruck der Pumpe ausreichend groß sein soll, um eine den Spalt teilweise bedeckende Luftblase in diesen hineinzusaugen um so ein Flüssigkeitsförderung zu ermöglichen, so lange der Spalt nicht vollständig mit einer Luftblase bedeckt ist, ist eine entsprechende Dimensionierung der Pumpe nur schwer zu realisieren. In diesem Zusammenhang ist daher entsprechend einer vorteilhaften Weiterbildung der erfindungsgemäßen Vorrichtung vorgesehen, daß zwischen dem Filter und der Pumpe ein weiteres Filter in Form eines Poren- und/oder Maschenfilters angeordnet ist, dessen Poren- bzw. Maschenweite geringer als die Breite des Spalts ist. Je nachdem, ob nun eine das Filter mit dem Spalt bedeckende Luftblase durch die Pumpe hindurchgefördert werden soll oder nicht, können die Pumpe und das Poren- und/oder Maschenfilter im Verhältnis zueinander dimensioniert werden. Solange das Filter mit dem Spalt nicht völlig von einer Luftblase bedeckt ist, wird diese an dem Spalt zurückgehalten und die zu pumpende Flüssigkeit durch den Spalt und anschließend durch das nachgeordnete Poren- bzw. Maschenfilter gefördert. An das Poren- der Maschenfilter gelangen daher nur vernachlässigbar kleine Luftbläschen, deren Durchmesser geringer als die Spaltbreite ist. Wenn der Spalt völlig mit einer Luftblase bedeckt ist, wird diese durch den Spalt hindurch gefördert und gelangt an das Poren- bzw. Maschenfilter. Je nach Poren- bzw. Maschengröße und der Größe des von der Pumpe erzeugten Saugdrucks bleibt die Luftblase an dem Poren- oder Maschenfilter hängen oder wird durch dieses hindurch gefördert.

An dieser Stelle soll nochmals auf den wesentlichen Unterschied zwischen dem Filter der erfindungsgemäßen Vorrichtung mit einem Spalt und einem herkömmlichen Poren- oder Maschenfilter hingewiesen werden, der darin besteht, daß bei dem Filter mit einem Spalt unabhängig vom Saugdruck der intermittierend arbeitenden Pumpe solange eine Luftblase mit größerem Durchmesser als der Spaltbreite zurückgehalten wird, wie die Luftblase den Spalt nicht völlig bedeckt; demgegenüber erfolgt bei einem Poren- oder Maschenfilter ein Luftdurchlaß, sobald bei einer Teilbedeckung des Filters mit Luft der Druckabfall der Flüssigkeitsströmung durch die unbedeckten Poren bzw. Maschen den Kapillardruck der Luftblasen über den von ihnen bedeckten Poren oder Maschen erreicht. Bei der erfindungsgemäßen Vorrichtung tritt daher insbesondere nicht der Fall ein, daß bei einer bestimmten Luftansammlung vor dem Filter ständig eine gewisse Luftmenge zusammen mit der zu pumpenden Flüssigkeit durch die Pumpe gefördert wird. Statt dessen ist bei der erfindungsgemäßen Vorrichtung bis zu dem Zeitpunkt, an dem die Luftansammlung vor dem Filter so groß geworden ist, daß dadurch der Spalt vollständig bedeckt wird, ein luftblasenfreier Flüssigkeitstransport sichergestellt.

Um bei einer vollständigen Luftbedeckung des Spalts geeignete Maßnahmen auslösen zu können, ist entsprechend einer vorteilhaften Weiterbildung der erfindungsgemäßen Vorrichtung vorgesehen, daß dem Filter eine Überwachungseinrichtung nachgeordnet ist, die Luftblasen in der durch das Filter hindurchgeförderten Flüssigkeit und/oder Abweichungen der Pumpfunktion von Sollwerten detektiert. Wenn die den Spalt bedeckende Luftblase durch das Filter gefördert wird, kann die Luftblase entweder direkt oder indirekt über Veränderungen in der Funktion der Pumpe detektiert werden. Bei der Infusion von Insulin kann die Detektion von Luft dem Patienten beispielsweise akustisch oder durch telemetrische Übertragung an ein vom Patienten getragenes Programmiergerät mitgeteilt werden, so daß dieser sich das Insulin so lange mit einer Spritze injizieren kann, bis die Pumpe wieder Insulin anstelle der Luft fördert; ferner kann der Patient ein Krankenhaus zur Entleerung und Wiederfüllung des Medikamentenspeichers aufsuchen. Soll dagegen das Pumpen von Luft von vorneherein ausgeschlossen werden, so kann durch die Überwachungseinrichtung die Pumpe automatisch ausgeschaltet werden. Wenn die den Spalt bedeckende Luftblase auf Grund des zu geringen Saugdrucks der Pumpe nicht durch den Spalt hindurchgefördert werden kann, so führt dies ebenfalls zu Änderungen der Pumpenfunktion oder sogar zum Pumpenstillstand. In der EP-A-0 317 705 sind Beispiele für solche Überwachungseinrichtungen beschrieben. So kann beispielsweise bei jeder einzelnen Pumpaktion die dabei verbrauchte Pumpenergie gemessen werden, die von den Verhältnissen im Flüssigkeitstrakt vor und hinter der Pumpe abhängig ist. Das gleiche gilt für das von der Pumpe bei jeder Pumpaktion erzeugte Pumpgeräusch oder bei einer Pumpe mit elektromagnetisch beschleunigtem Kolben für die Laufzeit des Kolbens von seiner Ruhestellung bis zum Erreichen der Endstellung. Schließlich kann das Vorhandensein von Luftblasen auch durch Messen der Lichtausbreitung, Schallausbreitung oder elektrischer Parameter in der zu pumpenden Flüssigkeit detektiert werden.

Zur Erläuterung der Erfindung wird im folgenden auf die Figuren der Zeichnung Bezug genommen; im einzelnen zeigen

FIG 1.    ein implantierbares Medikamentendosiergerät als bevorzugtes Ausführungsbeispiel für die erfindungsgemäße Vorrichtung mit einer Filteranordnung zum Zurückhalten von Luftblasen,

FIG 2.    ein Ausführungsbeispiel für die Filteranordnung in einer Schnittdarstellung und

FIG 3.    die Filteranordung in einer Ansicht von unten.

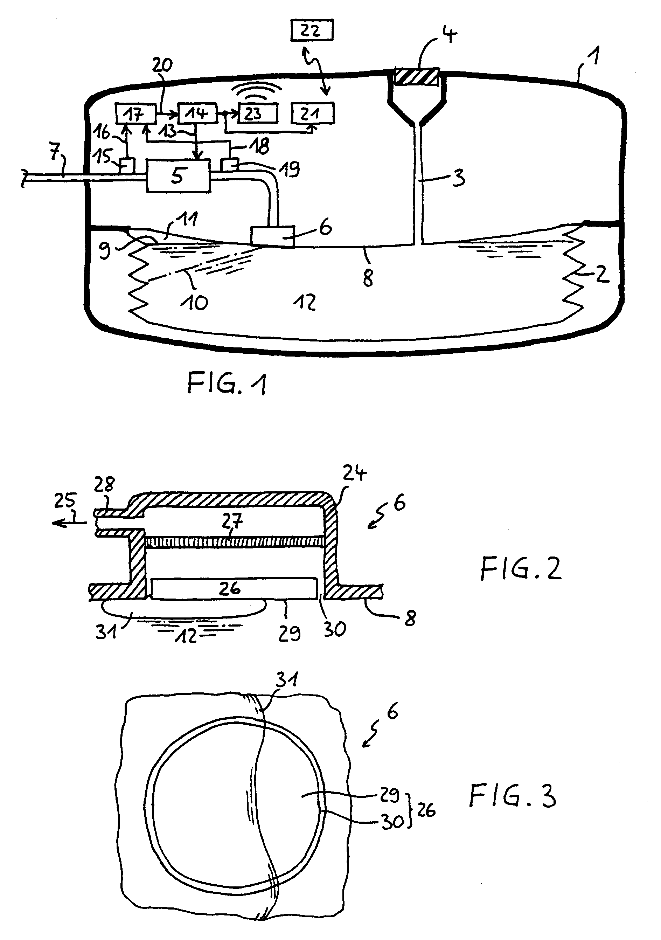

Das in Figur 1 gezeigte implantierbare Medikamentendosiergerät weist ein Gehäuse 1 auf, in dem ein Medikamentenspeicher 2 angeordnet ist. Eine Einlaßleitung 3 ist an einem Ende mit dem Inneren des Medikamentenspeichers 2 verbunden und an ihrem anderen Ende mit einem Einstichseptum 4 abgeschlossen, das in der Wand des Gehäuses 1 eingelassen ist und so das Innere des Medikamentenspeichers 2 gegenüber der Gehäuseumgebung abdichtet. Das Gehäuse 1 des Medikamentendosiergeräts wird in der Weise in dem Körper eines Patienten implantiert, daß das Einstichseptum 4 unter der Haut des Patienten zu liegen kommt, so daß ein flüssiges Medikament, beispielsweise Insulin, mit Hilfe einer Kanüle durch die Haut und das darunterliegende Einstichseptum 4 in den Medikamentenspeicher 2 einfüllbar ist. Eine Medikamentendosierpumpe 5 saugt das gespeicherte flüssige Medikament über eine Filteranordnung 6 in dosierten Portionen ab und führt diese einem Katheter 7 zu, der die Medikamentenportionen an eine geeignete Stelle im Körper des Patienten transportiert und dort injiziert.

Die Filteranordnung 6, deren Aufbau und Funktion unten näher an Hand von Figur 2 erläutert wird, dient zum Zurückhalten von Luftblasen, die als im Medikament gelöste Gase sowie beim Befüllen des Medikamentenspeichers 2 mit frischem Medikament in diesen gelangen können und sich dort ansammeln. Da vor jedem Wiederbefüllen des Medikamentenspeichers 2 dieser zunächst bis auf ein durch seinen Aufbau bedingtes Restvolumen entleert wird, kann durch eine geeignete Ausformung des Medikamentenspeichers 2 für jeden Wiederbefüllungsvorgang eine erlaubte Menge an zusammen mit dem Medikament in den Medikamentenspeicher 2 gelangender Luft definiert werden, wobei es unabhängig von der augenblicklichen Position des Medikamentendosiergeräts zu keiner völligen Luftbedeckung der Filteranordnung 6 kommt. Dazu ist bei dem gezeigten Ausführungsbeispiel die unmittelbar an die Filteranordnung 6 anschließende Innenwandung 8 des Medikamentenspeichers 2 konvex ausgebildet. Mit 9 und 10 sind für unterschiedliche Positionen des Medikamentendosiergeräts Grenzflächen zwischen einer in dem Medikamentenspeicher 2 enthaltenen bestimmten Luftmenge 11 und dem flüssigen Medikament 12 dargestellt, wobei es in keiner der beiden gezeigten Positionen zu einer völligen Luftbedekkung der Filteranordnung 6 kommt. Auf die Bedeutung hiervon wird untenstehend im Zusammenhang mit Figur 2 näher eingegangen.

Bei der Medikamentendosierpumpe 5 handelt es sich um eine intermittierend arbeitende Kolbenpumpe, wie sie beispielsweise aus der EP-A-0 259 668, EP-A-0 287 920 oder EP-A-0 317 705 bekannt ist. Eine solche Kolbenpumpe ist wegen des vernachlässigbaren Totraums in der Lage auch Luftblasen von einem Unterdruck auf der Ansaugseite der Pumpe 5 zu einem Überdruck am Ausgang der Pumpe 5 zu pumpen. Die Pumpe 5 ist über eine Steuerleitung 13 mit einer Steuereinrichtung 14 verbunden, die für jede auszuführende Pumpaktion eine Ansteuerung der Pumpe 5 bewirkt. Zur Überwachung der Pumpenfunktion ist im Bereich des Ausgangs der Pumpe 5 ein Geräuschsensor 15 angeordnet, der über eine Leitung 16 mit einer Überwachungsschaltung 17 verbunden ist. An der Überwachungsschaltung 17 ist ferner über eine weitere Leitung 18 ein Luftblasendetektor 19 beispielsweise in Form einer Reflexionslichtschranke angeschlossen, der auf der Ansaugseite der Pumpe 5 angeordnet ist. Die Überwachungsschaltung 17 wertet die Signale des Geräuschsensors 15 und des Luftblasendetektors 19 aus und führt sie über eine Steuerleitung 20 der Steuereinrichtung 14 zu, in der die Signale zur Pumpensteuerung herangezogen werden. Wenn von dem Luftblasendetektor 19 eine Luftblase detektiert wird und/oder auf Grund von Änderungen in den Pumpgeräuschen Fehlfunktionen der Pumpe 5 detektiert werden, wird das Störereignis über eine Telemetrieeinrichtung 21 an ein Programmiergerät 22 außerhalb des Körpers des Patienten übermittelt und/oder dem Patienten direkt durch einen Fehlermelder 23 akustisch mitgeteilt.

Figur 2 zeigt einen Schnitt durch die Filteranordnung 6 und Figur 3 eine Ansicht der Filteranordnung 6 auf ihrer dem Medikamentenspeicher 2 zugewandten Seite. Die Filteranordnung 6 weist ein auf der Seite zum Medikamentenspeicher 2 hin offenes Filtergehäuse 24 auf, in dem in Förderrichtung 25 der Pumpe 5 gesehen nacheinander ein Filter 26 und ein weiteres Filter 27 angeordnet sind. Hinter dem weiteren Filter 27 ist das Innere des Filtergehäuses 24 über eine Ausgangsleitung 28 mit der Ansaugseite der Pumpe 5 verbunden. Das Filter 26 verläuft auf seiner dem Inneren des Medikamentenspeichers 2 zugewandten Seite 29 bündig mit der Innenwand 8 des Medikamentenspeichers 2 und weist eine einzige Durchlaßöffnung in Form eines Ringspalts 30 auf. Das Filter 26 besteht aus reinem Titan, so daß es von der Medikamentenflüssigkeit 12 benetzt wird. Das dem Filter 26 nachgeordnete weitere Filter 27 ist als Poren- oder Maschenfilter aus einem von der Flüssigkeit 12 benetzbaren Material ausgebildet.

Der Dimensionierung des Spalts 30 liegen folgende Parameter zugrunde:

$Q_{max}$      maximale Flüssigkeitsströmung während eine Pumpaktion

V      Fördervolumen bei einer einzelnen Pumpaktion

$\sigma$      Oberflächenspannung der Flüssigkeit

$\eta$      Viskosität der Flüssigkeit

$\alpha$      Benetzungswinkel der Flüssigkeit

s      Spaltbreite

d      Spalttiefe

l      Spaltlänge

Um eine Luftblase 31 mit einem Durchmesser größer als der Spaltbreite s durch den Ringspalt 30 hindurchfördern zu können, ist eine Druckdifferenz $\Delta P_1$ über dem Spalt 30 mit

$$\Delta P_1 = (2{*}\sigma{*}\cos\alpha)/s$$

erforderlich. Bei der maximalen Flüssigkeitsströmung $Q_{max}$ ergibt sich über dem Filter 26 ein Druckabfall $\Delta P_2$ von

$$\Delta P_2 = 12{*}Q_{max}{*}\eta{*}d/(l{*}s^3),$$

wobei $\Delta P_2$ zunimmt, wenn der Spalt 30 teilweise mit Luft bedeckt ist, weil dadurch die effektive freie Spaltlänge abnimmt. Solange

$$\Delta P_1 > \Delta P_2$$

ist, kann keine Luftblase 31 mit einem Durchmesser größer als der Spaltbreite s den Spalt 30 passieren, so daß sich hieraus folgende Dimensionierungvorschrift für den Spalt 30 ergibt:

$$d/(l{*}s^2) < (\sigma{*}\cos\alpha)/(6{*}\eta{*}Q_{max}). \qquad (1)$$

Wenn der Spalt 30 fast völlig von der Luftblase 31 bedeckt ist, wird die freie Spaltlänge für den Flüssigkeitsdurchlaß so gering, daß

$$\Delta P_2 > \Delta P_1$$

wird und die Luftblase 31 in den Spalt 30 hineingezogen wird. Um zu verhindern, daß bei einer einzelnen Pumpaktion die Luftblase 31 den Spalt 30 passiert, ist das Spaltvolumen um einen Faktor $k_1$ ($k_1{>}1$) größer als das Fördervolumen V der Pumpe 5 bei einer Pumpaktion gewählt:

$$l{*}s{*}d > k_1{*}V \qquad (2)$$

Unter der Voraussetzung, daß der Spalt 30 an irgendeiner Stelle in Kontakt mit der Flüssigkeit 12 im Medikamentenspeicher 2 steht, kehrt unmittelbar nach der Pumpaktion die Grenzfläche zwischen der Luftblase 31 und der Flüssigkeit 12 auf Grund der Oberflächenspannung der Flüssigkeit 12 wieder in ihre Ausgangslage vor der Pumpaktion zurück, wobei sich der Spalt 30 wieder mit der Flüssigkeit 12 füllt, welche an der Stelle, an der der Spalt 30 in Kontakt mit der Flüssigkeit 12 steht aus dem Medikamentenspeicher 2 in den Spalt 30 gelangt.

Luftblasen mit einem Durchmesser kleiner als der Spaltbreite s können den Spalt 30 ungehindert passieren. Damit solche Luftblasen nur einen Bruchteil $k_2$ des Fördervolumens V der Pumpe 5 bei einer Pumpaktion ausmachen, wird für die Spaltbreite s folgende Dimensionierungsvorschrift gewählt:

$$s \leqq (6{*}k_2{*}V/\pi)^{1/3} \qquad (3)$$

Bei folgenden angenommenen Parameterwerten und gewählten Faktoren

$Q_{max} = 2{,}2{*}10^{-6}\ m^3/s$

$V = 10^{-9}\ m^3$

$\sigma = 0{,}06\ N/m$

$\eta = 0{,}7{*}10^{-3}\ Ns/m^2$

$\alpha = 0^0$

$k_1 = 2$

$k_2 = 10^{-3}$

werden die Dimensionierungsvorschriften (1),(2) und (3) beispielsweise durch folgende Dimensionierung des Spalts 30 erfüllt:

$$l \geqq 2{*}10^{-2}\ m,\ s = 10^{-4}\ m\ und\ d = 10^{-3}\ m.$$

Bei dieser Dimensionierung des Spaltes 30 ist ausgeschlossen, daß Luft den Spalt 30 passieren kann, solange nur der Spalt 30 an irgend einer Stelle Kontakt mit der Flüssigkeit 12 hat. Lediglich mikroskopisch kleine Luftblasen mit einem Durchmesser von weniger als $10^{-4}$ m und einem Volumen von weniger als 1/1000 des Fördervolumens V bei einer Pumpaktion können den Spalt 30 passieren. Wenn der Spalt 30 völlig von der Luftblase 31 bedeckt wird, ergibt sich ein Kapillardruck von $\Delta P_1 = 1200$ Pa, was ein vernachlässigbar geringer Druckabfall im Vergleich zu dem von der Pumpe 5 erzeugbaren Saugdruck ist. Die Pumpe 5 bleibt daher nicht stehen sondern pumpt solange Luft durch das Filter 26 bis der Spalt 30 wieder Kontakt mit der Flüssigkeit 12 bekommt.

Das weitere Filter 27 ist für den Fall vorgesehen, daß auch kleine Luftblasen mit einem Durchmesser kleiner als der Spaltbreite s zurückgehalten werden sollen. Bei einem Gesamtdurchmesser des weiteren Filters 27 von $10^{-2}$ m, einer Dicke von $150{*}10^{-6}$ m, einem Porendurchmesser von $10^{-5}$ m und einem 13%-igen Anteil der Poren an der Gesamtfläche des weiteren Filters 27 wird ein minimaler Druckunterschied $\Delta P_{max} =$

$2,4 \cdot 10^4$ Pa erzielt, um eine das weitere Filter 27 völlig bedeckende Luftblase durch dieses hindurchpumpen zu können; für den von der maximalen Flüssigkeitsströmung $Q_{max}$ hervorgerufenen Druckabfall über dem weiteren Filter 27 ergibt sich ein Wert von $7,3 \cdot 10^3$ Pa.

Wenn das weitere Filter 27 vollständig mit Luft bedeckt ist, können zwei Fälle eintreffen. Angenommen, daß bei einem Druck $P_R$ im Medikamentenspeicher 2 und einem Dampfdruck $P_V$ der Flüssigkeit 12 die Pumpe 5 in der Lage ist, einen den Dampfdruck $P_V$ erreichenden Saugdruck zu erzeugen, so wird die Luft durch das weitere Filter 27 hindurchgepumpt, wenn $P_R - P_V > \Delta P_{max}$.

Wenn dagegen $P_R - P_V < \Delta P_{max}$ gilt, oder der von der Pumpe 5 erzeugte Saugdruck nicht ausreicht, die Druckdifferenz $\Delta P_{max}$ zu überwinden, wird weder Flüssigkeit noch Luft durch das weitere Filter 27 hindurchgepumpt, so daß die Pumpe 5 lediglich Kavitationen erzeugt oder stehen bleibt.

Bei dem dargestellten Ausführungsbeispiel beträgt der Druck $P_R$ im Medikamentenspeicher 2 etwa $7 \cdot 10^4$ Pa und der Dampfdruck $P_V$ der Flüssigkeit 12 etwa $6,3 \cdot 10^3$ Pa, was dazu führt, daß bei einer völligen Luftbedeckung des weiteren Filters 27 die Pumpe 5 nicht stehen bleibt sondern die Luft durch das weitere Filter 27 hindurchpumpt.

Anstelle des dargestellten Ringspalts 30 können auch andere Spaltformen vorgesehen werden, wobei eine möglichst weite Längsausstreckung des Spalts anzustreben ist, um neben der Erzielung eines möglichst großen Spaltvolumens eine völlige Bedeckung des Spalts mit einer Luftblase weitestgehend zu verhindern. So kann der Spalt beispielsweise langgestreckt quer über die konvexe Innenwandung 8 des Medikamentenspeichers 2 verlaufen.

**Bezugszeichenliste**

| | |
|---|---|
| 1 | Gehäuse |
| 2 | Medikamentenspeicher |
| 3 | Einlaßleitung |
| 4 | Einstichseptum |
| 5 | Medikamentendosierpumpe |
| 6 | Filteranordnung |
| 7 | Katheter |
| 8 | konvexe Innenwandung von 2 |
| 9,10 | Grenzfläche zwischen 11 und 12 |
| 11 | Luft in 2 |
| 12 | flüssiges Medikament in 2 |
| 13 | Steuerleitung |
| 14 | Steuereinrichtung |
| 15 | Geräuschsensor |
| 16 | Leitung |
| 17 | Überwachungsschaltung |
| 18 | Leitung |
| 19 | Luftblasendetektor (Reflexionslichtschranke) |
| 20 | Steuerleitung |
| 21 | Telemetrieeinrichtung |
| 22 | Programmiergerät |
| 23 | Fehlermelder |
| 24 | Filtergehäuse |
| 25 | Förderrichtung von 12 |
| 26 | Filter |
| 27 | weiteres Filter |
| 28 | Ausgangsleitung |
| 29 | untere Seite von 26 |
| 30 | Ringspalt |
| 31 | Luftblase |

**Patentansprüche**

1. Vorrichtung zum Flüssigkeitstransport, insbesondere Medikamentendosiergerät, mit einer intermittierend arbeitenden Pumpe (5) und einem dieser eingangsseitig vorgeordneten Filter (26) zum Zurückhalten von Luftblasen (31), das derart dimensioniert ist, daß Luftblasen (31) mit einem größeren Durchmesser als ein vorgegebener Mindestdurchmesser an einem freien Passieren des Filters (26) gehindert werden und daß der bei einer Pumpaktion von der Flüssigkeitsströmung durch das Filter (26) erzeugte maximale Druckabfall geringer ist, als der zum Fördern einer Luftblase (31) mit einem größeren Durchmesser als der vorgegebene Mindestdurchmesser durch das Filter (26) erforderliche Druck, **dadurch gekennzeichnet**, daß das Filter (26) eine Durchlaßöffnung in Form eines langen Spalts (30) enthält und daß das Volumen des Spalts (30) größer als das Fördervolumen der Pumpe (5) bei einer einzelnen Pumpaktion ist.

2. Vorrichtung nach Anspruch 1, **dadurch gekennzeichnet**, daß das Filter (26) zumindest in der Umgebung des Spaltes (30) aus einem von der Flüssigkeit (12) benetzbaren Material besteht.

3. Vorrichtung nach Anspruch 1 oder 2, **dadurch gekennzeichnet**, daß der Flüssigkeit (12) ein Benetzungsmittel zugesetzt ist.

4. Vorrichtung nach einem der vorangehenden Ansprüche, **dadurch gekennzeichnet**, daß der Spalt (30) als Ringspalt ausgebildet ist.

5. Vorrichtung nach einem der vorangehenden Ansprüche, **dadurch gekennzeichnet**, daß das Filter (26) auf seiner von der Pumpe (5) abgewandten Seite (29) und/oder eine daran angrenzende Innenwandung (8) eines die zu pumpende Flüssigkeit (12) aufnehmenden Behälters (2) eine Ausformung aufweisen, die verhindert, daß eine Luftblase (11) im Bereich des Filters (26) eine stabile Gleichgewichtslage einnimmt.

6. Vorrichtung nach Anspruch 5, **dadurch gekennzeichnet**, daß das Filter (26) auf seiner von der Pumpe (5) abgewandten Seite (29) und/oder die daran angrenzende Innenwand (8) des Behälters (2) im wesentlichen konvex ausgebildet sind.

7. Vorrichtung nach einem der vorangehenden Ansprüche, **dadurch gekennzeichnet**, daß die Pumpe (5) eine Kolbenpumpe mit einem weitestgehend totraumfreien Hubraum ist.

8. Vorrichtung nach einem der vorangehenden Ansprüche, **dadurch gekennzeichnet**, daß zwischen dem Filter (26) und der Pumpe (5) ein weiteres Filter (27) in Form eines Poren- und/oder Maschenfilters angeordnet ist, dessen Poren- bzw. Maschenweite geringer als die Breite des Spaltes (30) ist.

9. Vorrichtung nach einem der vorangehenden Ansprüche, **dadurch gekennzeichnet**, daß dem Filter (26) eine Überwachungseinrichtung (15-19) nachgeordnet ist, die Luftblasen in der durch das Filter (26) hindurchgeförderten Flüssigkeit (12) und/oder Abweichungen der Pumpenfunktion von Sollwerten detektiert.

## Claims

1. Device for conveying liquid, in particular a medication dosing unit, with an intermittently operating pump (5), and with a filter (26) for retaining air bubbles (31) which is arranged upstream of the input side of this pump (5) and which is dimensioned in such a way that air bubbles (31) having a diameter greater than a predetermined minimum diameter are prevented from freely passing through the filter (26), and in that the maximum pressure drop generated, on a pump action, by the flow of liquid through the filter (26) is smaller than the pressure necessary for conveying an air bubble (31) having a diameter greater than the predetermined minimum diameter through the filter (26), characterized in that the filter (26) contains an admission opening in the form of a long gap (30), and in that the volume of the gap (30) is greater than the conveying volume of the pump (5) in a single pump action.

2. Device according to Claim 1, characterized in that the filter (26), at least in the area surrounding the gap (30), consists of a material which is wettable by the liquid (12).

3. Device according to Claim 1 or 2, characterized in that a wetting agent is added to the liquid (12).

4. Device according to one of the preceding claims, characterized in that the gap (30) is designed as an annular gap.

5. Device according to one of the preceding claims, characterized in that the filter (26) on its side (29) facing away from the pump (5) and/or an adjacent inner wall (8) of a reservoir (2) receiving the liquid (12) to be pumped is/are shaped so as to prevent an air bubble (11) from assuming a stable equilibrium position in the area of the filter (26).

6. Device according to Claim 5, characterized in that the filter (26) on its side (29) facing away from the pump (5) and/or the adjacent inner wall (8) of the reservoir (2) is/are essentially convex in design.

7. Device according to one of the preceding claims, characterized in that the pump (5) is a reciprocating pump having a piston capacity substantially free of dead space.

8. Device according to one of the preceding claims, characterized in that a further filter (27) in the form of a pore filter and/or mesh filter is arranged between the filter (26) and the pump (5), its pore width and/or mesh width being smaller than the width of the gap (30).

9. Device according to one of the preceding claims, characterized in that a monitoring means (15-19) is arranged downstream of the filter (26) and detects the air bubbles in the liquid (12) conveyed through the filter (26) and/or deviations of the pump function from set values.

## Revendications

1. Dispositif d'acheminement de liquide, notamment appareil de dosage de médicament, comportant une pompe (5) à fonctionnement intermittent et un filtre (26), monté en amont de l'entrée de celle-ci, destiné à retenir des bulles d'air (31) et dimensionné de telle manière que des bulles d'air (31), ayant un diamètre supérieur à un diamètre minimal prédéfini, ne puissent pas passer librement dans le filtre (26) et que, lors d'un fonctionnement de la pompe, la chute de pression maximale produite par l'écoulement de liquide dans le filtre (26) soit plus petite que la pression nécessaire à l'acheminement, à travers le filtre (26), d'une bulle d'air (31) ayant un diamètre supérieur au diamètre minimum prédéfini, caractérisé par le fait que le filtre (26) comporte une ouverture de passage sous la forme d'un long interstice (30), et que le volume de l'interstice (30) est plus grand que le volume de refoulement de la pompe (5) lors d'un seul actionnement de la pompe.

2. Dispositif selon la revendication 1, caractérisé par le fait que le filtre (26) consiste, du moins dans la zone entourant l'interstice (30), en un matériau mouillable par le liquide (12).

3. Dispositif selon la revendication 1 ou 2, caractérisé par le fait qu'un agent mouillant est ajouté au liquide (12).

4. Dispositif selon l'une des revendications précédentes, caractérisé par le fait que l'interstice (30) est réalisé sous la forme d'un interstice annulaire.

5. Dispositif selon l'une des revendications précédentes, caractérisé par le fait que le filtre (26), sur son côté (29) dirigé à l'opposé de la pompe (5) et/ou une paroi interne (8), immédiatement adjacente au filtre, d'un réservoir (2) contenant le liquide (12) à pomper présentent une forme empêchant qu'une bulle d'air (11), présente dans la zone du filtre (26) ne puisse prendre une position d'équilibre stable.

6. Dispositif selon la revendication 5, caractérisé par le fait que le filtre (26), sur son côté (29) dirigé à l'opposé de la pompe (5) et/ou la paroi interne (8), immédiatement adjacente au filtre, du réservoir (2) sont réalisés sensiblement convexes.

7. Dispositif selon l'une des revendications précédentes, caractérisé par le fait que la pompe (5) est une pompe à piston ayant une cylindrée exempte, dans la plus grande mesure possible, d'espace mort.

8. Dispositif selon l'une des revendications précédentes, caractérisé par le fait qu'on prévoit, entre le filtre (26) et la pompe (5), un filtre (27) supplémentaire sous la forme d'un filtre à pores et/ou à mailles, dont la taille de pore ou de maille est plus petite que la largeur de l'interstice (30).

9. Dispositif selon l'une des revendications précédentes, caractérisé par le fait que, en aval du filtre (26), est monté un dispositif de surveillance (15 à 19), qui détecte des bulles d'air dans le liquide (12) acheminé à travers le filtre (26), et/ou des écarts du fonctionnement de la pompe par rapport à des valeurs de consigne.

FIG. 1

FIG.2

FIG.3